# EUROPEAN PATENT SPECIFICATION

(11) **EP 3 946 569 B1**
(45) Date of publication and mention of the grant of the patent: **15.11.2023**
(21) Application number: 20722025.2
(22) Date of filing: 31.03.2020
(51) Int. Cl.: A61N 1/375

(54) **LOW-PROFILE CONTROL MODULE FOR AN ELECTRICAL STIMULATION SYSTEM**
STEUERMODUL MIT NIEDRIGEM PROFIL FÜR EIN ELEKTRISCHES STIMULATIONSSYSTEM
MODULE DE COMMANDE PEU ENCOMBRANT POUR UN SYSTÈME DE STIMULATION ÉLECTRIQUE

(30) Priority: 01.04.2019 US 201962827723 P
(43) Date of publication of application: 09.02.2022
(73) Proprietor: Boston Scientific Neuromodulation Corporation, Valencia, CA 91355 (US)
(72) Inventor: AGHASSIAN, Daniel, Glendale, CA 91208 (US)
(74) Representative: Vossius & Partner Patentanwälte Rechtsanwälte mbB
(86) International application number: PCT/US2020/025924
(87) International publication number: WO 2020/205843

(56) References cited:
- US-A1- 2004 176 816
- US-A1- 2004 267 332
- US-B1- 6 269 266
- US-B2- 9 555 242

## Description

### FIELD

The present invention is directed to the area of implantable electrical stimulation systems. The present invention is directed to a control module with an electronics housing and a power supply that form a low profile arrangement, as well as electrical stimulation systems that include the control module.

### BACKGROUND

Implantable electrical stimulation systems have proven therapeutic in a variety of diseases and disorders. For example, spinal cord stimulation systems have been used as a therapeutic modality for the treatment of chronic pain syndromes. Peripheral nerve stimulation has been used to treat chronic pain syndrome and incontinence, with a number of other applications under investigation. Functional electrical stimulation systems have been applied to restore some functionality to paralyzed extremities in spinal cord injury patients.

Stimulators have been developed to provide therapy for a variety of treatments. A stimulator can include a control module (with a pulse generator) and one or more stimulator electrodes. The one or more stimulator electrodes can be disposed along one or more leads, or along the control module, or both. The stimulator electrodes are in contact with or near the nerves, muscles, or other tissue to be stimulated. The pulse generator in the control module generates electrical pulses that are delivered by the electrodes to body tissue.

US 9,269,266 B1 discloses a power supply module for an implantable device that has a biocompatible outer housing which holds a repeatedly rechargeable electrochemical battery that supplies electrical power to a main module of the implantable device via a coupling element.

US 9,555,242 B1 discloses a cochlear implant module configured to be implanted within a patient and comprising cochlear implant circuitry configured to apply electrical stimulation representative of one or more audio signals to the patient, and a modular connector coupled to the cochlear implant module by way of a cable and configured to be implanted within the patient.

US 2004/0176816 A1 discloses a modular implantable medical device that includes two or more interconnected modules and an overmold that at least partially encapsulates each of the housings of the modules.

### BRIEF SUMMARY

The present invention relates to a control module for an electrical stimulation system according to claim 1. The control module includes an electronics housing; an electronic subassembly disposed within the electronics housing; a power source housing disposed adjacent to, and in contact with, the electronics housing; a power source disposed within the power source housing; and an interface comprising at least one first non-conductive feedthrough block and at least one interface feedthrough pin extending through each of the at least one first non-conductive feedthrough block, wherein the at least one first non-conductive feedthrough block is attached to one or more of the electronics housing or the power source housing, wherein the at least one interface feedthrough pin is electrically coupled to the power source and the electronic subassembly to provide power from the power source to the electronic subassembly; one or more connector assemblies extending laterally from the electronics housing, each of the one or more connector assemblies comprising a connector lumen configured and arranged to receive a lead, a plurality of connector contacts arranged along the connector lumen and in electrical communication with the electronic subassembly, and a plurality of connector conductors electrically coupled to the connector contacts; and a plurality of feedthrough pins extending through the electronics housing, wherein the conductors of the one or more connector assemblies are electrically coupled to the feedthrough pins and the feedthrough pins are electrically coupled to the electronic subassembly.

In at least some aspects, the first non-conductive feedthrough block is further attached to the power source housing. In at least some aspects, the interface further comprises a second non-conductive feedthrough block attached to the power source housing, wherein the at least one interface feedthrough pin extends through the second non-conductive interface.

In at least some aspects, the control module further includes an overmold disposed at least partially around the electronics housing and the power source housing. In at least some aspects, the overmold includes an opening through which the electronics housing is accessible so that the control module is configured, when attached to the patient, to contact patient tissue through the opening.

In at least some aspects, the control module further includes one or more connector assemblies, each of the one or more connector assemblies including a connector lumen configured and arranged to receive a lead, a plurality of connector contacts arranged along the connector lumen and in electrical communication with the electronic subassembly, and a plurality of connector conductors electrically coupled to the connector contacts. In at least some aspects, the control module further includes a plurality of feedthrough pins extending through the electronics housing, wherein the conductors of the one or more connector assemblies are electrically coupled to the feedthrough pins and the feedthrough pins are electrically coupled to the electronic subassembly. In at least some aspects, the control module further includes an overmold disposed at least partially around the electronics housing and the power source housing, wherein at least a portion of the connector assemblies extends outside of the overmold. In at least some aspects, the control module further includes an overmold disposed at least partially around the electronics housing and the power source housing, wherein the connector assemblies are disposed within the overmold.

In at least some aspects, the control module further includes a charging coil disposed external to the electronics housing and at least one feedthrough pin coupled to the charging coil, extending through the electronics housing, and coupled to the electronic subassembly. In at least some aspects, when the control module is configured for fastening to an outer surface of a skull, the control module extends radially outwards from the outer surface of the skull by an amount no greater than 7 mm. In at least some aspects, the electronics housing is hermetically sealed. In at least some aspects, the power source housing is hermetically sealed. In at least some aspects, the power source housing is not electrically coupled to the power source.

Moreover, a control module for an electrical stimulation system is disclosed. The control module includes an electronics housing; an electronic subassembly disposed within the electronics housing; a power source housing directly attached to the electronics housing; a power source disposed within the power source housing; and an interface between the electronics housing and the power source housing, the interface including at least two feedthrough pins that are electrically insulated from the electronics housing and the power source housing. The at least two feedthrough pins are electrically coupled to the power source and the electronic subassembly to provide power from the power source to the electronic subassembly.

In at least some aspects, the control module further includes an overmold disposed at least partially around the electronics housing and the power source housing. In at least some aspects, the overmold includes an opening through which the electronics housing is accessible so that the control module is configured, when attached to the patient, to contact patient tissue through the opening.

In at least some aspects, the control module further includes one or more connector assemblies, each of the one or more connector assemblies including a connector lumen configured and arranged to receive a lead, a plurality of connector contacts arranged along the connector lumen and in electrical communication with the electronic subassembly, and a plurality of connector conductors electrically coupled to the connector contacts. In at least some aspects, the control module further includes a plurality of feedthrough pins extending through the electronics housing, wherein the conductors of the one or more connector assemblies are electrically coupled to the feedthrough pins and the feedthrough pins are electrically coupled to the electronic subassembly.

In at least some aspects, the control module further includes a charging coil disposed external to the electronics housing and at least one feedthrough pin coupled to the charging coil, extending through the electronics housing, and coupled to the electronic subassembly.

In yet other aspects, an electrical stimulation system includes any of the control modules described above and an electrical stimulation lead coupleable to the control module. Optionally, the electrical stimulation system can further include a lead extension coupleable to the control module and the electrical stimulation lead.

### BRIEF DESCRIPTION OF THE DRAWINGS

Non-limiting and non-exhaustive embodiments of the present invention are described with reference to the following drawings. In the drawings, like reference numerals refer to like parts throughout the various figures unless otherwise specified.

For a better understanding of the present invention, reference will be made to the following Detailed Description, which is to be read in association with the accompanying drawings, wherein:
FIG. 1 is a schematic view of one embodiment of an electrical stimulation system;
FIG. 2 is a schematic side view of one embodiment of an electrical stimulation lead;
FIG. 3 is a schematic overview of one embodiment of components of a stimulation system, including an electronic subassembly disposed within a control module;
FIG. 4 is a schematic top view of one embodiment of a control module with an electronics housing adjacent a power source housing;
FIG. 5A is a schematic cross-sectional view of one embodiment of an interface and portions of an electronics housing and power source housing of a control module;
FIG. 5B is a schematic cross-sectional view of another embodiment of an interface and portions of an electronics housing and power source housing of a control module;
FIG. 5C is a schematic cross-sectional view of a third embodiment of an interface and portions of an electronics housing and power source housing of a control module;
FIG. 5D is a schematic cross-sectional view of a fourth embodiment of an interface and portions of an electronics housing and power source housing of a control module;
FIG. 6 is a schematic top view of another embodiment of a control module with an electronics housing adjacent a power source housing; and
FIG. 7 is a schematic top view of a third embodiment of a control module with an electronics housing adjacent a power source housing.

### DETAILED DESCRIPTION

The present invention is directed to the area of implantable electrical stimulation systems and methods of making and using the systems. The present invention is also directed to a control module with an electronics housing and a power supply that form a low profile arrangement, as well as electrical stimulation systems that include the control module and methods of making and using the control module.

Suitable implantable electrical stimulation systems include, but are not limited to, a least one lead with one or more electrodes disposed on a distal portion of the lead and one or more terminals disposed on one or more proximal portions of the lead. Leads include, for example, percutaneous leads, paddle leads, cuff leads, or any other arrangement of electrodes on a lead. Examples of electrical stimulation systems with leads are found in, for example, U.S. Patents Nos. 6,181,969; 6,516,227; 6,609,029; 6,609,032; 6,741,892; 7,244,150; 7,450,997; 7,672,734;7,761,165; 7,783,359; 7,792,590; 7,809,446; 7,949,395; 7,974,706; 8,175,710; 8,224,450; 8,271,094; 8,295,944; 8,364,278; 8,391,985; and 8,688,235; and U.S. Patent Applications Publication Nos. 2007/0150036; 2009/0187222; 2009/0276021; 2010/0076535; 2010/0268298; 2011/0005069; 2011/0004267; 2011/0078900; 2011/0130817; 2011/0130818; 2011/0238129; 2011/0313500; 2012/0016378; 2012/0046710; 2012/0071949; 2012/0165911; 2012/0197375; 2012/0203316; 2012/0203320; 2012/0203321; 2012/0316615; 2013/0105071; and 2013/0197602. In the discussion below, a percutaneous lead will be exemplified, but it will be understood that the methods and systems described herein are also applicable to paddle leads and other leads.

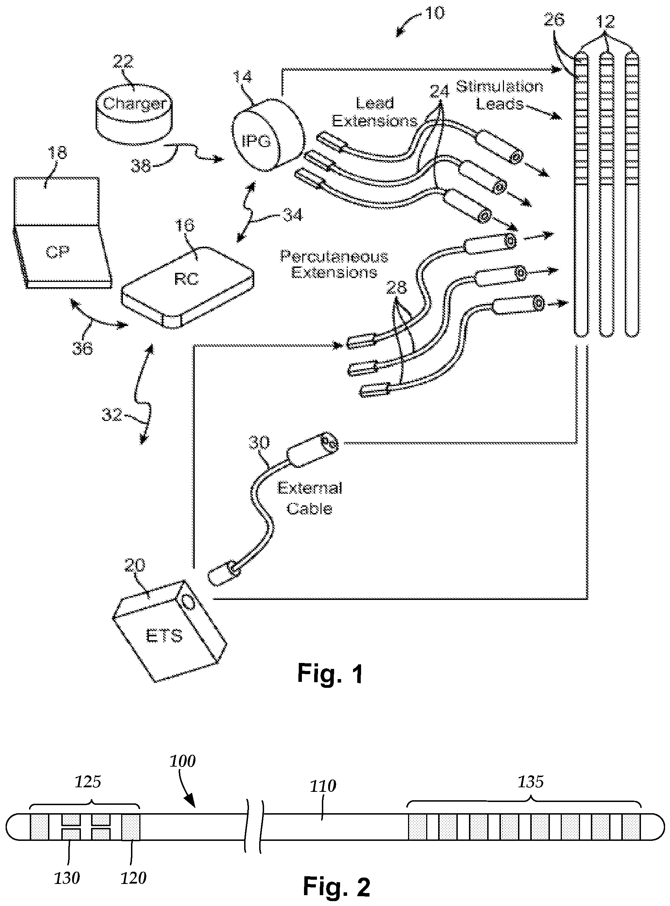

Turning to Figure 1, one embodiment of an electrical stimulation system 10 includes one or more stimulation leads 12 and an implantable pulse generator (IPG) 14. The system 10 can also include one or more of an external remote control (RC) 16, a clinician's programmer (CP) 18, an external trial stimulator (ETS) 20, or an external charger 22.

The IPG 14 is physically connected, optionally, via one or more lead extensions 24, to the stimulation lead(s) 12. Each lead carries multiple electrodes 26 arranged in an array. The IPG 14 includes pulse generation circuitry that delivers electrical stimulation energy in the form of, for example, a pulsed electrical waveform (i.e., a temporal series of electrical pulses) to the electrode array 26 in accordance with a set of stimulation parameters. The implantable pulse generator can have eight stimulation channels which may be independently programmable to control the magnitude of the current stimulus from each channel. In some embodiments, the implantable pulse generator can have more or fewer than eight stimulation channels (e.g., 4-, 6-, 16-, 32-, or more stimulation channels). The implantable pulse generator can have one, two, three, four, or more connector ports, for receiving the terminals of the leads and/or lead extensions.

The ETS 20 may also be physically connected, optionally via the percutaneous lead extensions 28 and external cable 30, to the stimulation leads 12. The ETS 20, which may have similar pulse generation circuitry as the IPG 14, also delivers electrical stimulation energy in the form of, for example, a pulsed electrical waveform to the electrode array 26 in accordance with a set of stimulation parameters. One difference between the ETS 20 and the IPG 14 is that the ETS 20 is often a non-implantable device that is used on a trial basis after the neurostimulation leads 12 have been implanted and prior to implantation of the IPG 14, to test the responsiveness of the stimulation that is to be provided. Any functions described herein with respect to the IPG 14 can likewise be performed with respect to the ETS 20.

The RC 16 may be used to telemetrically communicate with or control the IPG 14 or ETS 20 via a uni- or bi-directional wireless communications link 32. Once the IPG 14 and neurostimulation leads 12 are implanted, the RC 16 may be used to telemetrically communicate with or control the IPG 14 via a uni- or bi-directional communications link 34. Such communication or control allows the IPG 14 to be turned on or off and to be programmed with different stimulation parameter sets. The IPG 14 may also be operated to modify the programmed stimulation parameters to actively control the characteristics of the electrical stimulation energy output by the IPG 14. The CP 18 allows a user, such as a clinician, the ability to program stimulation parameters for the IPG 14 and ETS 20 in the operating room and in follow-up sessions. Alternately, or additionally, stimulation parameters can be programed via wireless communications (e.g., Bluetooth) between the RC 16 (or external device such as a hand-held electronic device) and the IPG 14.

The CP 18 may perform this function by indirectly communicating with the IPG 14 or ETS 20, through the RC 16, via a wireless communications link 36. Alternatively, the CP 18 may directly communicate with the IPG 14 or ETS 20 via a wireless communications link (not shown). The stimulation parameters provided by the CP 18 are also used to program the RC 16, so that the stimulation parameters can be subsequently modified by operation of the RC 16 in a stand-alone mode (i.e., without the assistance of the CP 18). The external charger 22 may charge a power source in the IPG 14 through a wireless link 38.

For purposes of brevity, the details of the RC 16, CP 18, ETS 20, and external charger 22 will not be further described herein. Details of exemplary embodiments of these devices are disclosed in U.S. Pat. No. 6,895,280. Other examples of electrical stimulation systems can be found at U.S. Patents Nos. 6,181,969; 6,516,227; 6,609,029; 6,609,032; 6,741,892; 7,949,395; 7,244,150; 7,672,734; and 7,761,165; 7,974,706; 8,175,710; 8,224,450; and 8,364,278; and U.S. Patent Application Publication No. 2007/0150036, as well as the other references cited above.

Turning to Figure 2, one or more leads are configured for coupling with a control module. The term "control module" is used herein to describe a pulse generator (e.g., the IPG 14 or the ETS 20 of Figure 1). Stimulation signals generated by the control module are emitted by electrodes of the lead(s) to stimulate patient tissue. The electrodes of the lead(s) are electrically coupled to terminals of the lead(s) that, in turn, are electrically coupleable with the control module. In some embodiments, the lead(s) couple(s) directly with the control module. In other embodiments, one or more intermediary devices (e.g., a lead extension, an adaptor, a splitter, or the like) are disposed between the lead(s) and the control module. The term "elongated member" used herein includes leads (e.g., percutaneous, paddle, cuff, or the like), as well as intermediary devices (e.g., lead extensions, adaptors, splitters, or the like). Percutaneous leads are described herein for clarity of illustration. It will be understood that paddle leads and cuff leads can be used in lieu of, or in addition to, percutaneous leads.

Figure 2 illustrates one embodiment of a lead 100 with electrodes 125 disposed at least partially about a circumference of the lead 100 along a distal end portion of the lead and terminals 135 disposed along a proximal end portion of the lead. The lead 100 can be implanted near or within the desired portion of the body to be stimulated such as, for example, the brain, spinal cord, or other body organs or tissues. In one example of operation for deep brain stimulation, access to the desired position in the brain can be accomplished by drilling a hole in the patient's skull or cranium with a cranial drill (commonly referred to as a burr), and coagulating and incising the dura mater, or brain covering. The lead 100 can be inserted into the cranium and brain tissue with the assistance of a stylet (not shown). The lead 100 can be guided to the target location within the brain using, for example, a stereotactic frame and a microdrive motor system. In some embodiments, the microdrive motor system can be fully or partially automatic. The microdrive motor system may be configured to perform one or more the following actions (alone or in combination): insert the lead 100, advance the lead 100, retract the lead 100, or rotate the lead 100.

In some embodiments, measurement devices coupled to the muscles or other tissues stimulated by the target neurons, or a unit responsive to the patient or clinician, can be coupled to the implantable pulse generator or microdrive motor system. The measurement device, user, or clinician can indicate a response by the target muscles or other tissues to the stimulation or recording electrode(s) to further identify the target neurons and facilitate positioning of the stimulation electrode(s). For example, if the target neurons are directed to a muscle experiencing tremors, a measurement device can be used to observe the muscle and indicate changes in, for example, tremor frequency or amplitude in response to stimulation of neurons. Alternatively, the patient or clinician can observe the muscle and provide feedback.

The lead 100 for deep brain stimulation can include stimulation electrodes, recording electrodes, or both. In at least some embodiments, the lead 100 is rotatable so that the stimulation electrodes can be aligned with the target neurons after the neurons have been located using the recording electrodes.

Stimulation electrodes may be disposed on the circumference of the lead 100 to stimulate the target neurons. Stimulation electrodes may be ring-shaped so that current projects from each electrode equally in every direction from the position of the electrode along a length of the lead 100. In the embodiment of Figure 2, two of the electrodes 125 are ring electrodes 120. Ring electrodes typically do not enable stimulus current to be directed from only a limited angular range around of the lead. Segmented electrodes 130, however, can be used to direct stimulus current to a selected angular range around the lead.

The lead 100 includes a lead body 110, terminals 135, and one or more ring electrodes 120 and one or more sets of segmented electrodes 130 (or any other combination of electrodes). The lead body 110 can be formed of a biocompatible, nonconducting material such as, for example, a polymeric material. Suitable polymeric materials include, but are not limited to, silicone, polyurethane, polyurea, polyurethane-urea, polyethylene, or the like. Once implanted in the body, the lead 100 may be in contact with body tissue for extended periods of time. In at least some embodiments, the lead 100 has a cross-sectional diameter of no more than 1.5 mm and may be in the range of 0.5 to 1.5 mm. In at least some embodiments, the lead 100 has a length of at least 10 cm and the length of the lead 100 may be in the range of 10 to 70 cm.

The electrodes 125 can be made using a metal, alloy, conductive oxide, or any other suitable conductive biocompatible material. Examples of suitable materials include, but are not limited to, platinum, platinum iridium alloy, iridium, titanium, tungsten, palladium, palladium rhodium, or the like. Preferably, the electrodes are made of a material that is biocompatible and does not substantially corrode under expected operating conditions in the operating environment for the expected duration of use.

Each of the electrodes can either be used or unused (OFF). When the electrode is used, the electrode can be used as an anode or cathode and carry anodic or cathodic current. In some instances, an electrode might be an anode for a period of time and a cathode for a period of time.

Deep brain stimulation leads may include one or more sets of segmented electrodes, with each set having electrodes circumferentially distributed about the lead at a particular longitudinal position. A set of segmented electrodes can include any suitable number of electrodes including, for example, two, three, four, or more electrodes.

Segmented electrodes may provide for superior current steering than ring electrodes because target structures in deep brain stimulation are not typically symmetric about the axis of the distal electrode array. Instead, a target may be located on one side of a plane running through the axis of the lead. Through the use of a radially segmented electrode array ("RSEA"), current steering can be performed not only along a length of the lead but also around a circumference of the lead. This provides precise three-dimensional targeting and delivery of the current stimulus to neural target tissue, while potentially avoiding stimulation of other tissue. Examples of leads with segmented electrodes include U.S. Patents Nos. 8,473,061; 8,571,665; and 8,792,993; U.S. Patent Application Publications Nos. 2010/0268298; 2011/0005069; 2011/0130803; 2011/0130816; 2011/0130817; 2011/0130818; 2011/0078900; 2011/0238129; 2012/0016378; 2012/0046710; 2012/0071949; 2012/0165911; 2012/0197375; 2012/0203316; 2012/0203320; 2012/0203321; 2013/0197424; 2013/0197602; 2014/0039587; 2014/0353001; 2014/0358208; 2014/0358209; 2014/0358210; 2015/0045864; 2015/0066120; 2015/0018915; 2015/0051681; U.S. Patent Applications Serial Nos. 14/557,211 and 14/286,797; and U.S. Provisional Patent Application Serial No. 62/113,291.

For illustrative purposes, the leads are described herein relative to use for deep brain stimulation, but it will be understood that any of the leads can be used for applications other than deep brain stimulation, including spinal cord stimulation, peripheral nerve stimulation, dorsal root ganglion stimulation, sacral nerve stimulation, or stimulation of other nerves, muscles, and tissues.

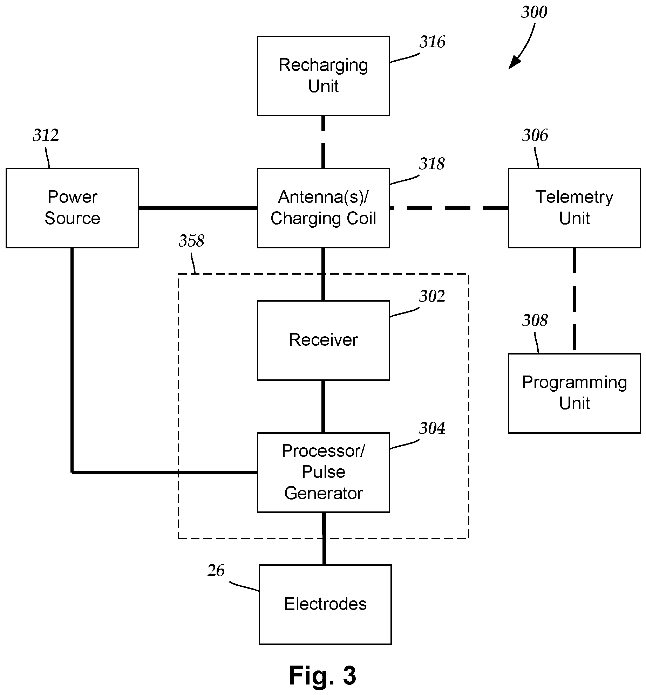

Figure 3 is a schematic overview of one embodiment of components of an electrical stimulation system 300 including a power source 312 and an electronic subassembly 358 which forms part of a control module, such as an IPG. The electronic subassembly 358 may include one or more components of the IPG. It will be understood that the electrical stimulation system can include more, fewer, or different components and can have a variety of different configurations including those configurations disclosed in the stimulator references cited herein. Some of the components (for example, a receiver 302 and a processor 304) of the electrical stimulation system can be positioned on one or more circuit boards or similar carriers within a sealed electronics housing of a control module, such as the IPG 14 in Figure 1, if desired.

Any power source 312 can be used including, for example, a battery such as a primary battery or a rechargeable battery. Examples of other power sources include super capacitors, nuclear or atomic batteries, mechanical resonators, infrared collectors, thermally-powered energy sources, flexural powered energy sources, bioenergy power sources, fuel cells, bioelectric cells, osmotic pressure pumps, and the like including the power sources described in U.S. Patent No. 7,437,193.

If the power source 312 is a rechargeable battery, the battery may be recharged using the optional antenna 318, if desired. Power can be provided to the battery for recharging by inductively coupling the battery through the antenna to a recharging unit 316 external to the user. Examples of such arrangements can be found in the references identified above. The electronic subassembly 358 and, optionally, the power source 312 can be disposed within a control module (e.g., the IPG 14 or the ETS 20 of Figure 1).

In one embodiment, electrical stimulation signals are emitted by the electrodes (e.g., 26 in Figure 1) to stimulate nerve fibers, muscle fibers, or other body tissues near the electrical stimulation system. The processor/pulse generator 304 is generally included to control the timing and electrical characteristics of the electrical stimulation system and to produce the electrical stimulation pulses. For example, the processor/pulse generator 304 can, if desired, control one or more of the timing, frequency, strength, duration, and waveform of the pulses. In addition, the processor 304 can select which electrodes can be used to provide stimulation, if desired. In some embodiments, the processor 304 selects which electrode(s) are cathodes and which electrode(s) are anodes. In some embodiments, the processor 304 is used to identify which electrodes provide the most useful stimulation of the desired tissue.

Any processor can be used and can be as simple as an electronic device that, for example, produces pulses at a regular interval or the processor can be capable of receiving and interpreting instructions from an external programming unit 308 that, for example, allows modification of pulse characteristics. In the illustrated embodiment, the processor 304 is coupled to a receiver 302 which, in turn, is coupled to the optional antenna 318. This allows the processor 304 to receive instructions from an external source to, for example, direct the pulse characteristics and the selection of electrodes, if desired.

In one embodiment, the antenna 318 is capable of receiving signals (*e.g.,* RF signals) from an external telemetry unit 306 which is programmed by the programming unit 308. (The same, or a different, antenna can be used for recharging the power supply 312.) The programming unit 308 can be external to, or part of, the telemetry unit 306. The telemetry unit 306 can be a device that is worn on the skin of the user or can be carried by the user and can have a form similar to a pager, cellular phone, or remote control, smart watch, if desired. As another alternative, the telemetry unit 306 may not be worn or carried by the user but may only be available at a home station or at a clinician's office. The programming unit 308 can be any unit that can provide information to the telemetry unit 306 for transmission to the electrical stimulation system 300. The programming unit 308 can be part of the telemetry unit 306 or can provide signals or information to the telemetry unit 306 via a wireless or wired connection. One example of a suitable programming unit is a computer operated by the user or clinician to send signals to the telemetry unit 306.

The signals sent to the processor 304 via the antenna 318 and the receiver 302 can be used to modify or otherwise direct the operation of the electrical stimulation system. For example, the signals may be used to modify the pulses of the electrical stimulation system such as modifying one or more of pulse duration, pulse frequency, pulse waveform, and pulse strength. The signals may also direct the electrical stimulation system 300 to cease operation, to start operation, to start charging the battery, or to stop charging the battery. In other embodiments, the stimulation system does not include the antenna 318 or receiver 302 and the processor 304 operates as programmed.

Optionally, the electrical stimulation system 300 may include a transmitter (not shown) coupled to the processor 304 and the antenna 318 for transmitting signals back to the telemetry unit 306 or another unit capable of receiving the signals. For example, the electrical stimulation system 300 may transmit signals indicating whether the electrical stimulation system 300 is operating properly or not or indicating when the battery needs to be charged or the level of charge remaining in the battery. The processor 304 may also be capable of transmitting information about the pulse characteristics so that a user or clinician can determine or verify the characteristics.

Conventional control modules include power sources, electronics, and connector assemblies that collectively create a size and shape that may limit the locations where the control module can be implanted or attached to the patient. At least some conventional control modules stack a battery above or below the main electronic subassembly, thereby forming a hermetic enclosure of a size or shape that limits potential implantation or attachment locations. In some instances, the size or shape of a control module may prevent the control module from physically fitting within a desired implantation or attachment location. In other instances, although a control module may physically fit within a desired implantation or attachment location, the size or shape of the control module may result in an undesirable cosmetic issue, such as the control module causing visible bulging.

In the case of deep brain stimulation, leads are typically extended through burr holes drilled into the patient's skull with the control module either implanted below the patient's clavicle area or disposed in a recessed region formed along an outer surface of the patient's skull. In the case of the former, the leads are undesirably tunneled along patient tissue from the burr holes in the skull to the patient's clavicle. In the case of the latter, a medical practitioner needs to carve out a section of skull large enough to position the control module within the carved-out region. Such a technique is time-consuming and tedious for the medical practitioner, and invasive for the patient.

As described herein, a low-profile control module can be implanted into, or otherwise attached to, a patient. The low-profile control module may increase the number of locations within a patient where a control module is implantable or attachable including the top portion of the skull. Furthermore, the control module may also improve patient cosmetics, by reducing undesirable bulging of patient tissue caused by the control module.

For illustrative purposes, the control module is described herein relative to use for deep brain stimulation. It will be understood, however, that the control module can be used for applications other than deep brain stimulation, including peripheral nerve stimulation (e.g., occipital nerve stimulation, pudental nerve stimulation, or the like), spinal cord stimulation, dorsal root ganglion stimulation, sacral nerve stimulation, or stimulation of other nerves, muscles, and tissues.

In at least some embodiments, the control module is suitable for disposing over the patient's skull and, optionally, beneath the patient's scalp. In at least some embodiments, the control module is attachable to an outer surface of the patient's skull without being inset into a recess carved into the skull. In at least some embodiments, when mounted to an outer surface of a patient's skull, the control module extends radially outwardly from the outer surface of the skull by no more than 20 mm, 18 mm, 16 mm, 14 mm, 12 mm, 10 mm, 9, mm, 8 mm, 7 mm, 6 mm, 5 mm, 4 mm, or 3 mm. Examples of attachment of a control module to the head or skull are illustrated in U.S. Patent Applications Nos. 16/186,058 and 16/358,174.

To maintain a relatively small footprint and height for the control module, an electronics housing, within which the electronic subassembly is disposed, and a power source housing, within which the power supply is disposed, are positioned adjacent to each other and in contact. An interface between the electronics housing and power source housing is used to couple the power supply to the electronic subassembly.

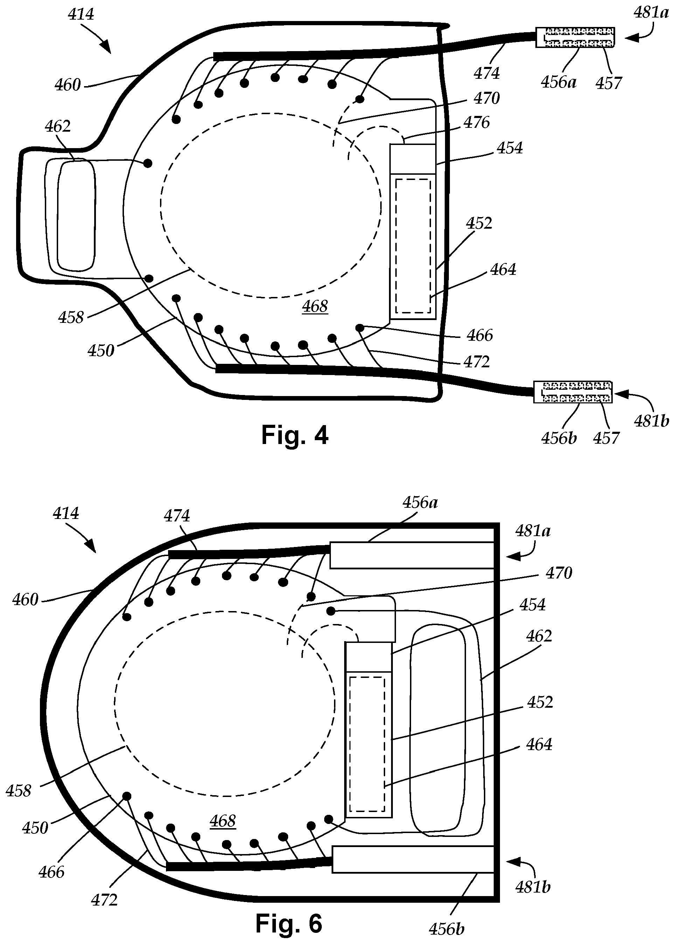

Figure 4 illustrates one embodiment of a control module 414 (for example, an IPG) which can be mounted, for example, on the head or skull of the patient. The control module 414 includes an electronics housing 450, a power source housing 452, an interface 454, two connector assemblies 456a, 456b, a charging coil 462, and an overmold 460. Within the electronics housing 450 is an electronic subassembly 458. In at least some embodiments the electronic subassembly 458 includes a printed circuit board arrangement. Within the power source housing 452 is a power source 464. In at least some embodiments, the power source housing 452 is a case-neutral housing as the power source housing is not electrically coupled to any of the terminals of the power source 464 (for example, there is a high impedance between the power source housing and the terminals of the power source.)

The electronics housing 450 and power source housing 452 are positioned adjacent to each other and, at least in some embodiments, are in contact with each other. This positioning of the electronics housing 450 and power source housing 452 provides a control module arrangement with a desirable combination of relatively small lateral area and relatively small height. In at least some embodiments, the electronics housing 450 and power source housing 452 can be welded or otherwise attached together or can be formed together (e.g., by molding or casting) in a unitary, integrated arrangement.

The electronics housing 450 forms a sealed cavity. In at least some embodiments, the power assembly housing 452 also forms a sealed cavity. In at least some embodiments, either, or both, of the sealed cavities is hermetically sealed.

The electronics housing 450 can be formed from any biocompatible material suitable for providing a sealed environment. In at least some embodiments, the electronics housing is formed from titanium or other metal material. In at least some embodiments, the electronics housing 450 is at least partially disc-shaped. In at least some embodiments, the electronics housing 450, alone or in combination with the overmold 460, has a height that is no more than 20 mm, 18 mm, 16 mm, 14 mm, 12 mm, 10 mm, 9, mm, 8 mm, 7 mm, 6 mm, 4 mm, 4 mm, or 3 mm.

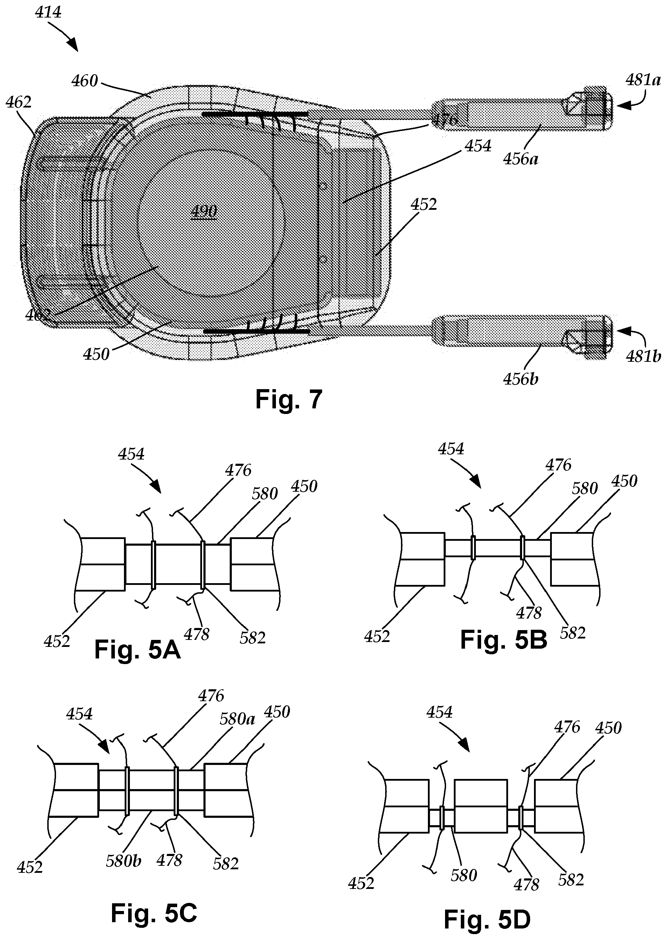

In at least some embodiments, multiple feedthrough pins, such as feedthrough pin 466, are disposed along an outer surface of the electronics housing 445. The feedthrough pins can be disposed at any suitable location along the outer surface of the electronics housing. In the illustrated embodiments, the feedthrough pins are disposed along the periphery of one major surface 468 of the electronics housing 450. The feedthrough pins 466 enable components external to the sealed cavity to electrically couple with the electronic subassembly 458 within the sealed cavity. The feedthrough pins 466 are electrically coupled to the electronic subassembly 458 (as shown schematically in Figure 4 by a dotted line 470) which may correspond to a wire, cable, trace on a circuit board, or any other suitable conductor. The feedthrough pins 466 are electrically insulated from the electronics housing 450. For example, the feedthrough pins 466 may be surrounded by a non-conductive block (for example, a ceramic block) that is brazed to the feedthrough pins 466 and the electronics housing 455. The non-conductive block and feedthrough pins can be the same as those discussed below with respective the interface 454, and illustrated in Figures 5A to 5D, except that the non-conductive blocks are brazed or otherwise attached to the electronics housing 450.

One or more connector assemblies 456a, 456b extend laterally from the electronics housing 450. In some embodiments, the control module includes multiple connector assemblies. In other embodiments, the control module includes a single connector assembly. The connector assemblies 456a, 456b are each configured and arranged to receive a single lead, although other assemblies might be configured to receive ends from multiple leads or multiple ends of a single lead. The connector assemblies 456a, 456b can extend outwardly from the electronics housing 450 in any suitable direction.

The connector assemblies 456a, 456b define connector lumens 481a, 481b, respectively. Each connector assembly 456a, 456b is configured to receive a proximal portion of a lead. An array of connector contacts 457 is arranged along each of the connector lumens 481a, 481b, respectively, and is configured to electrically couple with terminals of the leads when the proximal portions of the leads are received by the connector assemblies. The connector contacts 457 can be electrically isolated from one another by electrically-nonconductive spacers (not shown). The connector assemblies may, optionally, include end stops to promote alignment of the lead terminals with the connector contacts. Examples of suitable connector assemblies include, but are not limited to, those described in U.S. Patent Application Publications Nos. 2015/0209575; 2016/0059019; 2016/0129265; 2009/0233491; 2009/0264943; 2017/0014635; 2017/0072187; 2017/0143978; 2017/0203104; 2018/0028820; 2017/0361108; 2018/0008832; 2018/0093098; 2018/0214687; 2018/0243570; 2018/0289968; 2018/0369596; 2019/0030345; 2019/0083794; 2019/0083793; and U.S. Patent Applications Nos. 16/149,868 and 16/223,745.

The connector contacts 457 are electrically coupled to the electronic subassembly 458. In at least some embodiments connector conductors 472 (which may be collected into a cable 474 or other arrangement) can be extended between the connector contacts 457 and the feedthrough pins 466 disposed along the electronics housing 450 which, in turn, extend into the electronics housing and electrically couple with the electronic subassembly 458.

In at least some embodiments, an optional overmold 460 is disposed over at least a portion of each of the electronics housing 450 and the power source housing 452 to provide protection to the control module. In at least some embodiments, the overmold 460 may also protect skin or other tissue of the patient or may soften corners of the electronics housing 450 or power source housing 452. In at least some embodiments, the overmold 460 also seals at least a portion of each of the electronics housing, power assembly, and one or more connector assemblies. In at least some embodiments, the overmold 460 is formed from an electrically-nonconductive material to insulate the electrical components from one another and/or the patient. The covering can be formed from any suitable biocompatible material. In at least some embodiments, the overmold 460 is formed from silicone, polyurethane, or other suitable polymer material. In at least some embodiments, one or more of the overmold 460, the electronics housing 450, or the power source housing 452 is configured for fastening to the patient (for example, to the skull of the patient) using fasteners, such as sutures, staples, screws, or the like. For example, there may be fastening apertures in one or more of the overmold 460, the electronics housing 450, or the power source housing 452 for receiving the fasteners.

In at least some embodiments, the control module 414 includes a charging coil 462. In at least some embodiments, the charging coil 462 is coupled to the electronic subassembly 458 (or the power source 464) through one or more of the feedthrough pins 466.

In at least some embodiments, the control module 414 includes one or more antennas (e.g., Bluetooth, or the like) which may include the charging coil 462 or be separate from the charging coil. In at least some embodiments, the charging coil and antenna(s) are disposed external to the electronics housing. In at least some embodiments, the charging coil and antenna(s) are disposed beneath, or embedded within, the overmold 460.

In at least some embodiments, the power source 464 is electrically coupled to the electronic subassembly 458 through an interface 454. Figures 5A to 5C illustrate, in cross-section, examples of different interfaces 454. It will be understood, however, that other interface arrangements can be used. In Figures 5A to 5C, the electronics housing 450 and power source housing 452 are adjacent to each other. The interface 454 includes a non-conductive feedthrough block 580 with at least two interface feedthrough pins 582 passing through the non-conductive feedthrough block 580. The non-conductive feedthrough block 580 can be, for example, a ceramic block that is brazed or otherwise attached to one, or both, of the electronics housing 450 or power source housing 452. The non-conductive feedthrough block 580 will be surrounded along its perimeter by one, or both, of the electronics housing 450 or power source housing 452 to provide a seal. In the interface 454 of Figure 5A, the non-conductive feedthrough block 580 is attached to both the electronics housing 450 and the power source housing 452.

In the interface 454 of Figure 5B, the non-conductive feedthrough block 580 is attached to only the electronics housing 450. In other embodiments, the non-conductive feedthrough block 580 can be attached only to the power supply housing. The interface 454 of Figure 5C has two individual non-conductive feedthrough blocks 580a, 580b which are attached to the electronics housing 450 and power source housing 452, respectively. In the interface 454 of Figure 5D, the non-conductive feedthrough block 580 is attached to only the power source housing 450 and there is a separate non-conductive feedthrough block 580 associated with each interface feedthrough pin 582. Individual or separated non-conductive feedthrough blocks 580 can be utilized in any of the other embodiments of Figures 5A to 5D. Any number of non-conductive feedthrough blocks 580 can be used. Any number of interface feedthrough pins 582 can extend through a non-conductive feedthrough block. Any other suitable arrangement, or combination of any of the illustrated arrangements, can be used.

The interface feedthrough pins 582 can be made of any suitable material including, but not limited to, titanium, gold, platinum, or the like and can have any suitable shape or configuration. The interface feedthrough pins 582 are coupled to conductors 476 that attach to the electronic subassembly 458 (Figure 4) and conductors 478 that attach to the power source 464 (Figure 4). The conductors 476, 478 can be independently wires, cables, traces on a circuit board, or any other suitable conductors. The interface feedthrough pins 582 are electrically insulated from the electronics housing 450 and the power source housing 452.

Figure 6 illustrates another embodiment of a control module 414 that is similar to the control module of Figure 4 except that the charging coil 462 is in a different position and the connector assemblies 456a, 456b are disposed in the overmold 160.

Figure 7 is a bottom view of another embodiment of a control module. In this embodiment, the interface 454 lies along a lateral side of the power source housing 452 instead of at one end of the power source housing as in the embodiments of Figures 4 and 6. In addition, Figure 7 illustrates an optional opening 790 in the overmold 460 to permit tissue contact with the electronics housing 450. Such contact can permit the electronics housing 450 to act as a remote electrode (e.g., a return electrode) for electrical stimulation. This optional opening 790 can also be used with the control modules illustrated in Figures 4 and 6.

The above specification and examples provide a description of the manufacture and use of the invention. Since many embodiments of the invention can be made without departing from the scope of the invention, the invention also resides in the claims hereinafter appended.

## Claims

1. A control module (414) for an electrical stimulation system, the control module comprising:
an electronics housing (450);
an electronic subassembly (358, 458) disposed within the electronics housing;
a power source housing (452) disposed adjacent to, and in contact with, the electronics housing;
a power source (312, 464) disposed within the power source housing; and
an interface (454) comprising at least one first non-conductive feedthrough block (580, 580a) and at least one interface feedthrough pin (582) extending through each of the at least one first non-conductive feedthrough block, wherein the at least one first non-conductive feedthrough block is attached to one or more of the electronics housing or the power source housing, wherein the at least one interface feedthrough pin is electrically coupled to the power source and the electronic subassembly to provide power from the power source to the electronic subassembly;
**characterized by**
one or more connector assemblies (456a, 456b) extending laterally from the electronics housing, each of the one or more connector assemblies comprising
a connector lumen (481a, 481b) configured and arranged to receive a lead,
a plurality of connector contacts (457) arranged along the connector lumen and in electrical communication with the electronic subassembly, and
a plurality of connector conductors (472) electrically coupled to the connector contacts; and
a plurality of feedthrough pins (466) extending through the electronics housing, wherein the conductors of the one or more connector assemblies are electrically coupled to the feedthrough pins and the feedthrough pins are electrically coupled to the electronic subassembly.

2. The control module of claim 1, wherein the first non-conductive feedthrough block is further attached to the power source housing.

3. The control module of claim 1, wherein the interface further comprises a second non-conductive feedthrough block (580b) attached to the power source housing, wherein the at least one interface feedthrough pin extends through the second non-conductive feedthrough block.

4. The control module of claim 1, wherein the power source housing is directly attached to the electronics housing.

5. The control module of any one of claims 1-4, further comprising an overmold (460) disposed at least partially around the electronics housing and the power source housing.

6. The control module of any one of claims 1-5, wherein the overmold comprises an opening (490) through which the electronics housing is accessible so that the control module is configured, when attached to the patient, to contact patient tissue through the opening.

7. The control module of any one of claims 1-6, wherein the one or more connector assemblies is a plurality of the connector assemblies.

8. The control module of claim 1, further comprising an overmold disposed at least partially around the electronics housing and the power source housing, wherein at least a portion of the connector assemblies extends outside of the overmold.

9. The control module of claim 1, further comprising an overmold disposed at least partially around the electronics housing and the power source housing, wherein the connector assemblies are disposed within the overmold.

10. The control module of any one of claims 1-9, further comprising a charging coil (462) disposed external to the electronics housing and at least one feedthrough pin coupled to the charging coil, extending through the electronics housing, and coupled to the electronic subassembly.

11. The control module of any one of claims 1-10, wherein, when the control module is configured for fastening to an outer surface of a skull, the control module extends radially outwards from the outer surface of the skull by an amount no greater than 7 mm.

12. The control module of any one of claims 1-11, wherein the electronics housing is hermetically sealed.

13. The control module of claim 12, wherein the power source housing is hermetically sealed.

14. The control module of any one of claims 1-13, wherein the power source housing is not electrically coupled to the power source.

15. An electrical stimulation system comprising:
the control module of any one of claims 1-14; and
an electrical stimulation lead (12) coupleable to the control module.

## Patentansprüche

1. Steuermodul (414) für ein elektrisches Stimulationssystem, wobei das Steuermodul aufweist:
ein Elektronikgehäuse (450);
eine elektronische Unteranordnung (358, 458), die in dem Elektronikgehäuse angeordnet ist;
ein Energiequelle-Gehäuse (452), das angrenzend an das und in Kontakt mit dem Elektronikgehäuse angeordnet ist;
eine Energiequelle (312, 464), die in dem Energiequelle-Gehäuse angeordnet ist; und
eine Schnittstelle (454) mit mindestens einem ersten nichtleitfähigen Durchführungsblock (580, 580a) und mindestens einem Schnittstelle-Durchführungsstift (582), der sich durch jeden des mindestens einen ersten nichtleitfähigen Durchführungsblocks erstreckt, wobei der mindestens eine erste nichtleitfähige Durchführungsblock an dem Elektronikgehäuse und/oder dem Energiequelle-Gehäuse angebracht ist, wobei der mindestens eine Schnittstelle-Durchführungsstift mit der Energiequelle und der elektronischen Unteranordnung elektrisch gekoppelt ist, um der elektronischen Unteranordnung Energie aus der Energiequelle bereitzustellen;
**gekennzeichnet durch**
eine oder mehrere Anschlussanordnungen (456a, 456b), die sich lateral des Elektronikgehäuses erstreckt/erstrecken, wobei jede der einen oder mehreren Anschlussanordnungen aufweist:
ein Anschlusslumen (481a, 481b), das konfiguriert und angeordnet ist, einen Leiter zu empfangen,
mehrere Anschlusskontakte (457), die entlang des Anschlusslumens angeordnet sind und in elektrischer Verbindung mit der elektronischen Unteranordnung sind, und
mehrere Anschlussleiter (472), die mit den Anschlusskontakten elektrisch gekoppelt sind; und
mehrere Durchführungsstifte (466), die sich durch das Elektronikgehäuse erstrecken, wobei die Leiter der einen oder mehreren Anschlussanordnungen mit den Durchführungsstiften elektrisch gekoppelt sind und die Durchführungsstifte mit der elektronischen Unteranordnung elektrisch gekoppelt sind.

2. Steuermodul nach Anspruch 1, wobei der erste nichtleitfähige Durchführungsblock ferner an dem Energiequelle-Gehäuse angebracht ist.

3. Steuermodul nach Anspruch 1, wobei die Schnittstelle ferner einen zweiten nichtleitfähigen Durchführungsblock (580b) aufweist, der an dem Energiequelle-Gehäuse angebracht ist, wobei der mindestens eine Schnittstelle-Durchführungsstift sich durch den zweiten nichtleitfähigen Durchführungsblock erstreckt.

4. Steuermodul nach Anspruch 1, wobei das Energiequelle-Gehäuse direkt an dem Elektronikgehäuse angebracht ist.

5. Steuermodul nach einem der Ansprüche 1 bis 4, ferner aufweisend eine Umspritzung (460), die zumindest teilweise um das Elektronikgehäuse und das Energiequelle-Gehäuse angeordnet ist.

6. Steuermodul nach einem der Ansprüche 1 bis 5, wobei die Umspritzung eine Öffnung (490) aufweist, durch die das Elektronikgehäuse zugänglich ist, so dass das Steuermodul, wenn es an dem Patienten angebracht ist, konfiguriert ist, durch die Öffnung Patientengewebe zu kontaktieren.

7. Steuermodul nach einem der Ansprüche 1 bis 6, wobei die eine oder mehreren Anschlussanordnungen eine Vielzahl der Anschlussanordnungen ist/sind.

8. Steuermodul nach Anspruch 1, ferner aufweisend eine Umspritzung, die zumindest teilweise um das Elektronikgehäuse und das Energiequelle-Gehäuse angeordnet ist, wobei mindestens ein Teil der Anschlussanordnungen sich außerhalb der Umspritzung erstreckt.

9. Steuermodul nach Anspruch 1, ferner aufweisend eine Umspritzung, die zumindest teilweise um das Elektronikgehäuse und das Energiequelle-Gehäuse angeordnet ist, wobei die Anschlussanordnungen in der Umspritzung angeordnet sind.

10. Steuermodul nach einem der Ansprüche 1 bis 9, ferner aufweisend eine außerhalb des Elektronikgehäuses angeordnete Ladespule (462) und mindestens einen Durchführungsstift, der mit der Ladespule gekoppelt ist, sich durch das Elektronikgehäuse erstreckt und mit der elektronischen Unteranordnung gekoppelt ist.

11. Steuermodul nach einem der Ansprüche 1 bis 10, wobei, wenn das Steuermodul konfiguriert ist, an einer Außenfläche eines Schädels befestigt zu werden, das Steuermodul sich von der Außenfläche des Schädels um einen Betrag von nicht größer als 7 mm radial auswärts erstreckt.

12. Steuermodul nach einem der Ansprüche 1 bis 11, wobei das Elektronikgehäuse hermetisch versiegelt ist.

13. Steuermodul nach Anspruch 12, wobei das Energiequelle-Gehäuse hermetisch versiegelt ist.

14. Steuermodul nach einem der Ansprüche 1 bis 13, wobei das Energiequelle-Gehäuse mit der Energiequelle nicht elektrisch gekoppelt ist.

15. Elektrisches Stimulationssystem aufweisend:
das Steuermodul nach einem der Ansprüche 1 bis 14; und
eine elektrische Stimulationsleitung (12), die an das Steuermodul koppelbar ist.

## Revendications

1. Module de commande (414) pour un système de stimulation électrique, le module de commande comprenant :
un boîtier d'électronique (450) ;
un sous-ensemble électronique (358, 458) disposé au sein du boîtier d'électronique ;
un boîtier de source d'électricité (452) disposé de manière adjacente au boîtier d'électronique et en contact avec celui-ci ;
une source d'électricité (312, 464) disposée au sein du boîtier de source d'électricité ; et
une interface (454) comprenant au moins un premier bloc de traversée non conducteur (580, 580a) et au moins une broche de traversée d'interface (582) s'étendant à travers chacun de l'au moins un premier bloc de traversée non conducteur, dans lequel l'au moins un premier bloc de traversée non conducteur est fixé sur l'un ou plusieurs parmi le boîtier d'électronique ou le boîtier de source d'électricité, dans lequel l'au moins une broche de traversée d'interface est électriquement couplée à la source d'électricité et au sous-ensemble électronique pour fournir de l'électricité, de la source d'électricité au sous-ensemble électronique ;
**caractérisé par** un ou plusieurs ensembles de connecteur (456a, 456b) s'étendant latéralement à partir du boîtier d'électronique, chacun des un ou plusieurs ensembles de connecteur comprenant
une lumière de connecteur (481a, 481b) configurée et agencée pour recevoir un fil,
une pluralité de contacts de connecteur (457) agencés le long de la lumière de connecteur et en communication électrique avec le sous-ensemble électronique, et
une pluralité de conducteurs de connecteur (472) électriquement couplés aux contacts de connecteur ; et
une pluralité de broches de traversée (466) s'étendant à travers le boîtier d'électronique, dans lequel les conducteurs des un ou plusieurs ensembles de connecteur sont électriquement couplés aux broches de traversée et les broches de traversée sont électriquement couplées au sous-ensemble électronique.

2. Module de commande selon la revendication 1, dans lequel le premier bloc de traversée non conducteur est en outre fixé sur le boîtier de source d'électricité.

3. Module de commande selon la revendication 1, dans lequel l'interface comprend en outre un second bloc de traversée non conducteur (580b) fixé sur le boîtier de source d'électricité, dans lequel l'au moins une broche de traversée d'interface s'étend à travers le second bloc de traversée non conductrice.

4. Module de commande selon la revendication 1, dans lequel le boîtier de source d'électricité est directement fixé sur le boîtier d'électronique.

5. Module de commande selon l'une quelconque des revendications 1 à 4, comprenant en outre un surmoulage (460) disposé au moins partiellement autour du boîtier d'électronique et du boîtier de source d'électricité.

6. Module de commande selon l'une quelconque des revendications 1 à 5, dans lequel le surmoulage comprend une ouverture (490) à travers laquelle le boîtier d'électronique est accessible de sorte que le module de commande soit configuré, lorsqu'il est fixé sur le patient, pour être en contact avec un tissu du patient à travers l'ouverture.

7. Module de commande selon l'une quelconque des revendications 1 à 6, dans lequel les un ou plusieurs ensembles de connecteur sont une pluralité des ensembles de connecteur.

8. Module de commande selon la revendication 1, comprenant en outre un surmoulage disposé au moins partiellement autour du boîtier d'électronique et du boîtier de source d'électricité, dans lequel au moins une partie des ensembles de connecteur s'étend à l'extérieur du surmoulage.

9. Module de commande selon la revendication 1, comprenant en outre un surmoulage disposé au moins partiellement autour du boîtier d'électronique et du boîtier de source d'électricité, dans lequel les ensembles de connecteur sont disposés au sein du surmoulage.

10. Module de commande selon l'une quelconque des revendications 1 à 9, comprenant en outre une bobine de charge (462) disposée de manière externe au boîtier d'électronique et au moins une broche de traversée couplée à la bobine de charge, s'étendant à travers le boîtier d'électronique, et couplée au sous-ensemble électronique.

11. Module de commande selon l'une quelconque des revendications 1 à 10, dans lequel, lorsque le module de commande est configuré pour être attaché sur une surface externe d'un crâne, le module de commande s'étend radialement vers l'extérieur à partir de la surface externe du crâne d'une quantité non supérieure à 7 mm.

12. Module de commande selon l'une quelconque des revendications 1 à 11, dans lequel le boîtier d'électronique est hermétiquement scellé.

13. Module de commande selon la revendication 12, dans lequel le boîtier de source d'électricité est hermétiquement scellé.

14. Module de commande selon l'une quelconque des revendications 1 à 13, dans lequel le boîtier de source d'électricité n'est pas électriquement couplé à la source d'électricité.

15. Système de stimulation électrique comprenant :
le module de commande selon l'une quelconque des revendications 1 à 14 ; et
un fil de stimulation électrique (12) apte à être couplé au module de commande.
